# EUROPEAN PATENT SPECIFICATION

(11) **EP 2 647 346 B1**
(45) Date of publication and mention of the grant of the patent: **18.01.2017**
(21) Application number: 11844634.3
(22) Date of filing: 17.11.2011
(51) Int. Cl.: A61B 18/18

(54) **MICROWAVE SURGICAL TOOL**
CHIRURGISCHES MIKROWELLENINSTRUMENT
INSTRUMENT CHIRURGICAL À MICRO-ONDES

(30) Priority: 30.11.2010 JP 2010266401
(43) Date of publication of application: 09.10.2013
(73) Proprietor: Orient Microwave Corp., Shiga 527-0135 (JP); Shiga University Of Medical Science, Otsu-shi, Shiga 520-2192 (JP)
(72) Inventor: TANI, Tohru, Otsu-shi Shiga 520-2192 (JP); KITAMURA, Masatoshi, Higashiomi-shi Shiga 527-0135 (JP); ONO, Akiyoshi, Higashiomi-shi Shiga 527-0135 (JP); KAWAMURA, Hideaki, Higashiomi-shi Shiga 527-0135 (JP); SAITO, Shigeru, Higashiomi-shi Shiga 527-0135 (JP)
(74) Representative: Vossius & Partner Patentanwälte Rechtsanwälte mbB
(86) International application number: PCT/JP2011/076501
(87) International publication number: WO 2012/073709

(56) References cited:
- WO-A1-2010/092328
- JP-A- 3 092 182
- JP-A- 2003 523 799
- JP-A- 2007 535 370
- JP-A- 2008 237 627
- JP-A- 2010 192 421
- US-A1- 2010 145 328

## Description

### Technical Field

The present invention relates to a microwave surgical instrument.

### Background Art

Patent Document 1 discloses an example of a known microwave surgical instrument for emitting a microwave to a biological tissue so as to coagulate the tissue or stanch blood. This microwave surgical instrument is composed of a microwave-generating unit, and a surgical electrode for irradiating a biological tissue with a microwave generated from the microwave-generating unit. The microwave surgical instrument performs coagulation, blood stanching, incision, or the like with respect to a biological tissue in a biological body using dielectric heat generated by the irradiation of the biological tissue with a microwave from the surgical electrode.

### Citation List

### Patent Document

PTD 1: Japanese Patent Publication No. 3782495

. Further medical microwave systems are known from documents US2010/0145328 A1 and WO2010/092328 A1.

### Summary of Invention

### Technical Problem

In the above microwave surgical instrument, the microwave-generating unit and the surgical electrode are connected through a coaxial cable via connectors. The coaxial cable transmits a microwave from the microwave-generating unit to the surgical electrode. However, the power loss of the coaxial cable is very large; the transmitting efficiency is about 30 to 50%. The transmitting efficiency further decreases due to impedance mismatch in the biological tissue. To compensate for such a great power loss in the coaxial cable, it is necessary to use a high-power microwave-generating unit. This poses a problem in regards to the necessity of increasing the size of the microwave-generating unit. In view of this problem, an object of the present invention is to provide a microwave surgical instrument that can be decreased in size.

### Solution to Problem

The invention is defined in the appended set of claims.

The microwave surgical instrument according to the present invention is composed of a surgical instrument main body having an electrode section for emitting a microwave to a biological tissue; a microwave oscillator, internally provided in the surgical instrument main body, for oscillating a microwave; and an amplifier, internally provided in the surgical instrument main body by being connected between the electrode section and the microwave oscillator, for amplifying a microwave from the microwave oscillator, and transmitting the microwave to the electrode section.

In hitherto-known microwave instruments, a microwave-generating unit having a microwave oscillator and an amplifier is separated from the surgical instrument main body. Therefore, the microwave-generating unit and the surgical instrument main body are connected (more specifically, the amplifier and the electrode section are connected) through a long, flexible coaxial cable of about 2 to 3 m. However, the microwave surgical instrument of the present invention is structured such that the microwave-generating unit having a microwave oscillator and an amplifier is internally provided in the surgical instrument main body. In this structure, unlike in the hitherto-known instruments, it is not necessary to connect the electrode section and the amplifier with a long, flexible coaxial cable. Therefore, the electrode section and the amplifier can be connected with an inflexible coaxial cable of, for example, about 1 to 15 cm, more preferably about 10 to 14 cm, thereby reducing power loss. Thus, it becomes unnecessary to ensure high power for the microwave-generating unit, allowing the microwave surgical instrument to be decreased in size. This also enables the entire body of the microwave surgical instrument to be decreased in size. Further, although hitherto-known microwave surgical instruments are stationarily installed because of their large bodies, the microwave surgical instrument according to the present invention, which can thus be decreased in size, can be used as a portable instrument; therefore, the microwave surgical instrument of the present invention can be used as a mobile surgical instrument. Additionally, such an advantage also solves the problem of insufficient operability of the surgical instrument main body due to insufficient flexibility of the flexible coaxial cable. More specifically, as mentioned above, the microwave surgical instrument of the present invention is structured such that the microwave oscillator and the amplifier are internally provided in the surgical instrument main body; therefore, the coaxial cable for connecting the surgical instrument main body and the microwave-generating unit is not required. Thereby, the present invention improves the operability of the surgical instrument main body.

Further, the microwave-generating unit having the microwave oscillator and the amplifier may also be structured as a semiconductor microwave-generating unit containing a semiconductor element as means for generating and amplifying a microwave. In the hitherto-known instruments, the microwave-generating unit is made of a magnetron containing a ferromagnetic substance so as to compensate for the power loss in the coaxial cable. However, since the microwave surgical instrument of the present invention does not contain a coaxial cable, such compensation of power loss is not necessary. Thus, the microwave-generating unit can be structured using a semiconductor element. This microwave-generating unit containing a semiconductor element does not contain a ferromagnetic substance; thus, the microwave-generating unit can be used with an MRI device.

Further, the microwave-generating unit may also include a variable output matching circuit, provided between the amplifier and the electrode section, for matching output impedance of the amplifier and impedance of the biological tissue; a detection circuit for separately detecting a reflected power and a incident power between the amplifier and the electrode section; and controlling means for controlling the variable output matching circuit based on the reflected power and the incident power detected by the detection circuit. Because biological tissues are subject to a great change in electromagnetic impedance, the reflected power returning to the microwave-generating unit is also increased; thus, the microwave irradiation power efficiency is about 10% to 20%. On the other hand, as described above, by controlling the variable output matching circuit based on the incident power and the reflected power, it is possible to match the variable impedance of biological tissues and the output impedance of the amplifier, thereby increasing the microwave irradiation power efficiency. Additionally, in the hitherto-known instruments, a protection device such as an isolator containing a ferromagnetic substance is provided to prevent damage of the microwave-generating unit due to synthesis of the reflected power and the incident power. However, the above structure does not require a protection device such as an isolator. Therefore, the above microwave-generating unit can be used with an MRI device.

Further, the microwave-generating unit may further include a low-frequency constant current source for supplying a low-frequency alternating current to the electrode section. With this structure, it is possible to apply a low-frequency alternating current to a biological tissue, allowing monitoring of changes in electric resistance in a biological tissue. According to the fact that a change in biological tissue caused by microwave irradiation changes the electric resistance in the tissue, it is possible to, for example, determine completion of blood stanching when the resistance value is decreased by about 30 to 50%. The "low frequency" is not limited insofar as there is no influence of electrolysis of, H₂O, Na ion or the like in a biological tissue. For example, the frequency is preferably about 500 Hz to 10 kHz. The waveform is preferably rectangular.

Additionally, the microwave-generating unit may further include a housing for storing an electronic circuit section having a microwave oscillator, an amplifier, etc.; and a cooling water bag, provided near the housing, into which cooling water is supplied. With this structure, it is possible to efficiently release the heat from the housing by the cooling water.

Further, by adopting a structure in which the amount of cooling water to be supplied to the cooling water bag is adjusted according to the timing of microwave irradiation, it is possible to more efficiently release the heat.

Additionally, a physiological saline solution may be used as cooling water. In this case, a structure having a water-discharging path for discharging the physiological saline solution to the electrode section may be provided. This structure enables the discharged solution to be used to wash the electrode section, thereby preventing adhesion of carbonized tissues to the electrode section, and preventing temperature increase in the surrounding tissues.

The surgical instrument main body preferably further includes an insertion unit having an electrode section on its end. The insertion unit is preferably detachable from the main body. With this structure, it becomes possible to immerse only the insertion unit in an antiseptic solution after the insertion unit is detached from the surgical instrument main body; i.e., it is possible to immerse only the insertion unit, without immersing the surgical instrument main body containing an electronic circuit section.

### Advantageous Effects of Invention

The present invention provides a mobile microwave surgical instrument that can be decreased in size, and thus can be easily carried.

### Brief Description of Drawings

[Fig. 1] Fig. 1 is a front view of a microwave surgical instrument according to the present embodiment.
[Fig. 2] Fig. 2 is a bottom view of a microwave surgical instrument according to the present embodiment.
[Fig. 3] Figs. 3 (a) and 3(b) are a front view (a) and a lateral view (b) of a housing of the present embodiment.
[Fig. 4] Fig. 4 is a lateral view of a housing according to another embodiment.
[Fig. 5] Fig. 5 is a lateral view of a housing provided with a cooling water bag according to the present embodiment.
[Fig. 6] Fig. 6 is a plan view of Fig. 5.
[Fig. 7] Fig. 7 is a lateral view of a housing provided with a cooling water bag according to another embodiment.
[Fig. 8] Fig. 8 is a circuit diagram showing an electronic circuit section according to the present embodiment.
[Fig. 9] Fig. 9 is a lateral view of a housing provided with a cooling water bag according to another embodiment.

### Description of Embodiments

Hereunder, an embodiment of a microwave surgical instrument according to the present invention is described in reference to attached drawings.

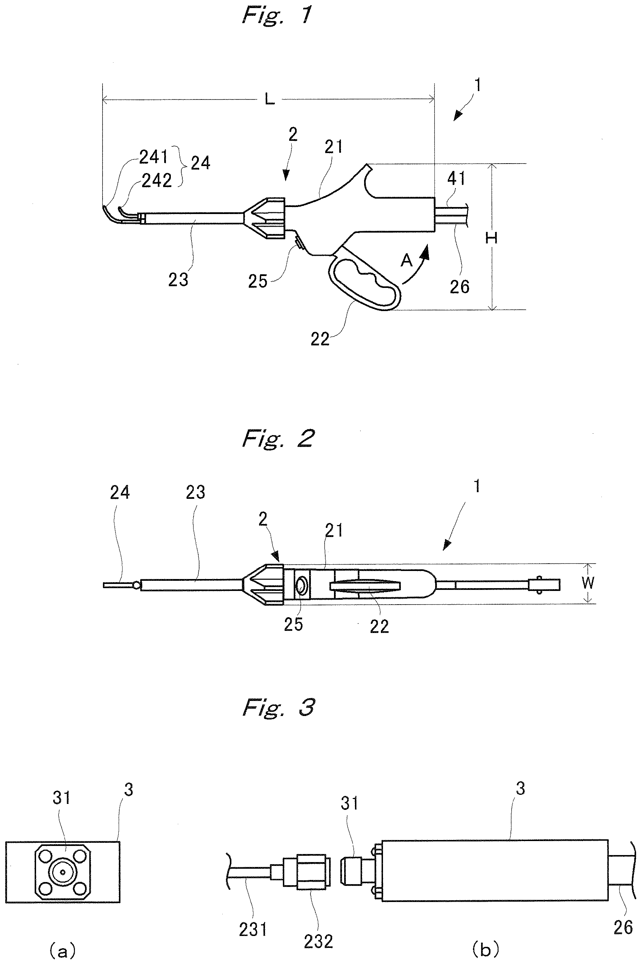

As shown in Fig. 1 and Fig. 2, a microwave surgical instrument 1 includes a surgical instrument main body 2 having an electrode section 24 on its top end. The surgical instrument main body 2 is mainly composed of a main body grip 21, a slide grip 22 swingably attached to the main body grip 21, and an insertion unit 23 detachably mounted on the top end of the main body grip 21. During the operation, the insertion unit 23 is inserted into a human body; therefore, a biological tissue or blood is more easily adhered to the insertion unit 23. On the top end of the insertion unit 23, an electrode section 24 is provided.

The electrode section 24 is composed of a first electrode 241 and a second electrode 242. The first electrode 241 and the second electrode 242 are structured such that they come closer to each other by the movement of the slide grip 22 toward the main body grip 21 as indicated by the arrow A, allowing them to pinch a biological tissue. The second electrode 242 serves to supply a microwave, and the first electrode 241 serves as a GND electrode, which is a return electrode. The main body grip 21 includes a switch 25 for turning on and off the microwave irradiation. By pressing the switch 25, a microwave is emitted from the electrode section 24. The microwave irradiation is stopped by releasing the switch 25.

At the back end of the main body grip 21, a power supply cable 26 for supplying power to an electronic circuit section 5 (described later) and a water supply tube 41 for supplying cooling water for releasing the heat from the electronic circuit section 5 extend outward. The power supply cable 26 and the water supply tube 41 are connected or connectable to an electric source or a cooling water supply. The entire length (the length from the top end of the electrode section 24 to the back end of main body grip 21) L of the surgical instrument main body 2 is about 250 to 300 mm. The height H of the surgical instrument main body 2 when the slide grip 22 is most distant from the main body grip 21 is about 25 to 30 mm. The width W of the surgical instrument main body 2 is about 120 to 140 mm. However, the length, the height, and the width are not limited to the above ranges.

Inside the surgical instrument main body 2, more specifically, inside the main body grip 21, a rectangular housing 3 is provided as shown in Fig. 3. Although it is not particularly limited, the housing 3 can be formed of aluminum or the like in view of aluminum's light weight and excellent heat conduction. The housing 3 contains an electronic circuit section 5 (described later) including a microwave oscillator 51, an amplifier 52, a variable output matching circuit 53, a detection circuit 54, a microcontroller 55, and the like. The microcontroller 55 may be stored as a separate unit in another portion in the surgical instrument main body 2 (in particular, in the main body grip 21) instead of being stored in the housing 3. In this case, the microcontroller 55 may be connected with the above members provided inside the housing 3 via connectors.

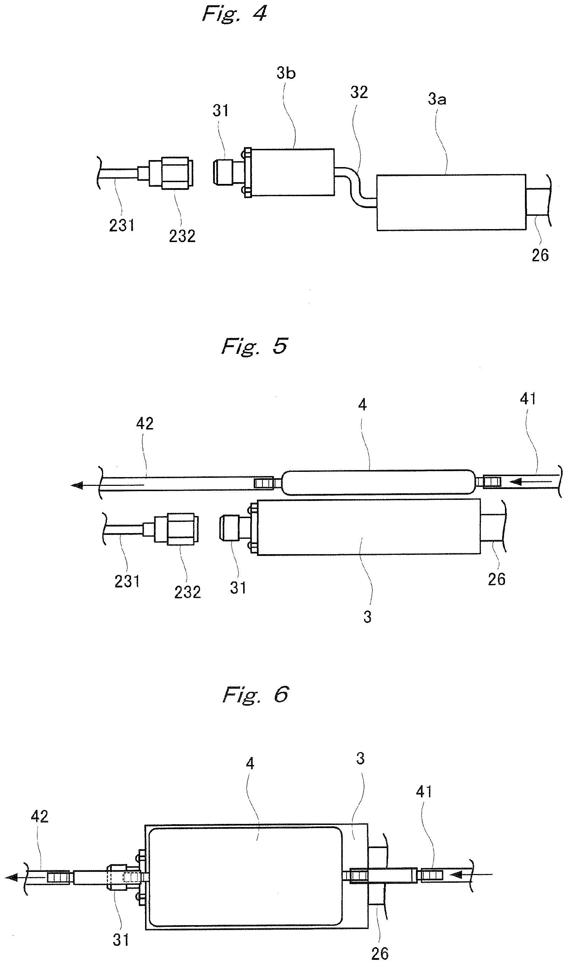

On the top end of the housing 3, a connector 31 made of an SMA connector or the like is provided. By screwing the insertion unit 23 into the main body grip 21, the connector 31 is connected with a connector 232 provided on the end of a feed line 231 in the insertion unit 23. The feed line 231 is formed of, for example, an inflexible coaxial low-loss cable having a length of about 1 to 15 cm, more preferably about 10 to 14 cm. Further, when the inner portion of the main body grip 21 has a complex shape, the housing 3 may be provided as two separate portions for easier installation. For example, as shown in Fig. 4, a first housing 3a containing the microwave oscillator 51 and the amplifier 52, and a second housing 3b containing the variable output matching circuit 53, the detection circuit 54, and the like may be provided. Since the size of the microcontroller 55 can be greatly decreased (for example, about 2×2×1 cm) by incorporating the majority thereof into a microcomputer chip, the microcontroller 55 may be stored in an extra space in the main body grip 21 or inside the housing 3. When the microcontroller 55 is stored in the housing 3, it may be stored both in the housing 3a and in the housing 3b. In this case, the electronic circuit in the first housing 3a and the electronic circuit in the second housing 3b are connected via an inflexible coaxial low-loss cable 32 having a length of about 1 to 15 cm, more preferably about 10 to 14 cm, or a control signal line (not shown).

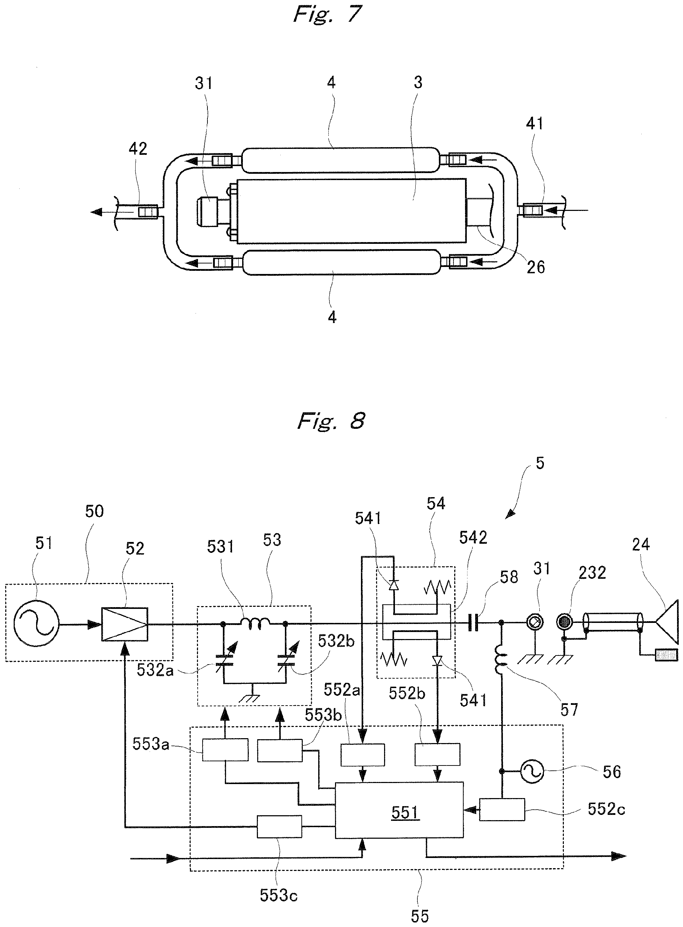

To prevent problems such as a decrease in microwave power or unstable operation, it is necessary to effectively release the heat generated in the electronic circuit section 5 in the housing 3. Therefore, in the present embodiment, as shown in Fig. 5 and Fig. 6, a cooling water bag 4 is provided above the housing 3 by covering nearly the entire upper surface of the housing 3. The cooling water bag 4 is connected to an external cooling water supply (not shown) via the water supply tube 41 so as to supply cooling water thereto. The cooling water supply may be, for example, an infusion solution bag filled with a physiological saline solution. The water in the cooling water bag 4 that absorbs heat from the housing 3 is externally discharged through the water discharge tube 42. The water discharged from the water discharge tube 42 is supplied to the electrode section 24, thereby washing the electrode section 24. This prevents adhesion of the carbonized portion to the electrode section 24, and also prevents an increase in temperature of the surrounding tissues. The material of the cooling water bag 4 is not particularly limited insofar as it is capable of transferring heat from the housing 3 to the physiological saline solution. Examples of the materials include polyethylene terephthalate (PET). As shown in Fig. 7, the cooling water bag 4 may also be provided below the housing 3 as well as above the housing 3 so as to more efficiently release the heat. In this case, the water supply tube 41 and the water discharge tube 42 may be structured such that each of them are individually branched into the two cooling water bags 4; or such that two water supply tubes 41 and two water discharge tubes 42 are separately connected to the two cooling water bags 4.

Next, the electronic circuit section 5 provided inside the housing 3 is described below in detail. The electronic circuit section 5 is composed of surface mount devices, and it is integrally provided in its entirety as microstrip lines or the like on a dielectric substrate.

As shown in Fig. 8, the electronic circuit section 5 includes a microwave-generating unit 50 composed of a microwave oscillator 51, and an amplifier 52 for amplifying microwaves. The microwave oscillator 51 may be a known microwave oscillator composed of a semiconductor element such as a GaAs MES field effect transistor. The amplifier 52, which amplifies microwaves oscillated from the microwave oscillator 51, may be, for example, formed of a high-efficiency GaN field effect transistor suitable for a high-power device.

Further, during the surgery, the impedance of the biological tissue greatly changes depending on the way of applying the blade edge of the surgical instrument or thermal changes in the tissues. Therefore, if the amplifier 52 and the electrode section 24 are directly connected, the output impedance of the microwave-generating unit 50 (in particular, the amplifier 52) and the impedance of the biological tissue do not match, and the reflected power increases. Consequently, the efficiency with which the microwave energy is absorbed into the biological tissue decreases. In the present embodiment, the variable output matching circuit 53 is provided so as to perform, at first, the impedance matching between the microwave-generating unit 50 (in particular, amplifier 52) and the electrode section 24. The variable output matching circuit 53 includes an inductor 531, the first and second variable capacitors 532a and 532b, and performs impedance matching by adjusting the electrostatic capacities of the first and second variable capacitors 532a and 532b, thereby minimizing the reflected power. The variable capacitors 532a and 532b are not limited insofar as they are capable of adjusting the electrostatic capacities, such as a high-voltage varactor diode (variable capacitance diode).

The electrostatic capacities of the variable capacitors 532a and 532b are determined based on the incident power and the reflected power between the microwave-generating unit 50 (in particular, amplifier 52) and the electrode section 24. To enable this control, the electronic circuit section 5 includes the detection circuit 54 and the microcontroller 55. The detection circuit 54 is connected between the variable output matching circuit 53 and the electrode section 24, and is mainly composed of a directional wave detector 541 and a bidirectional coupler 542. The microcontroller 55 is mainly composed of a microprocessor 551 for performing calculation or control, analogue/digital converters (ADC) 552a to 552c, digital/analogue converters (DAC) 553a to 553c, a memory (not shown), and the like.

A method for controlling the electrostatic capacities of the variable capacitors 532a and 532b by the detection circuit 54 and the microcontroller 55 is described below. The detection circuit 54 detects the incident power and the reflected power between the microwave-generating unit 50 (in particular, amplifier 52) and the electrode section 24, and the microcontroller 55 controls the electrostatic capacities of variable capacitors 532a and 532b based on the detected data. More specifically, first, a microwave signal detected by the bidirectional coupler 542 is supplied to the wave detector 541. The microwave signal thus supplied to the wave detector 541 is converted into a direct current voltage according to its power level by the wave detector 541. The resulting voltage is converted into a digital signal by the analogue/digital converters 552a and 552b, and the resulting signal is supplied to the microprocessor 551. The microprocessor 551 performs calculation based on the incident power and the reflected power transmitted from the detection circuit 54 to find control data of the variable capacitor for ensuring a possible maximum Pi/Pr (a ratio of the incident power Pi to the reflected power Pr). The control data is converted into an analogue signal (direct current voltage) by the digital/analogue converters 553a and 553b to control the electrostatic capacities of the first and second variable capacitors 532a and 532b of the variable output matching circuit 53. This series of controls is repeated to maintain the maximum Pi/Pr, i.e., a so-called feedback control is performed.

In the electronic circuit section 5, the low-frequency constant current source 56 is connected to an electrode section 24 via a high-frequency choke coil (RFC) 57. The low-frequency constant current source 56 supplies a low-frequency alternating current of a constant value to a biological tissue via an electrode section 24. Due to the provision of a capacitor 58, the low-frequency constant current is supplied only to the electrode section 24. As such, the completion of the blood stanching of the biological tissue can be determined by this operation of supplying the low-frequency alternating current by the low-frequency constant current source 56. More specifically, when the blood stanching is completed, the resistance is changed (specifically, the resistance is decreased by about 30 to 50%); thus, the completion of the blood stanching of the biological tissue can be determined by the change in resistance. More specifically, the determination is performed by capturing the amplitude value Vs of the low-frequency alternating voltage and the amplitude value Ic of the low-frequency constant current into the microprocessor 551 via an analogue/digital converter 552c. The amplitude values are converted into a resistance Rs according to Rs=Vs/Ic.

When the resistance Rs is decreased by about 30 to 50%, the microprocessor 551 causes the microwave-generating unit 50 (in particular, amplifier 52) to stop microwave oscillation via the digital/analogue converter 553c. The microcontroller 55 can also cause the microwave-generating unit 50 (in particular, amplifier 52) to apply microwave power according to the data regarding changes in resistance or the optimal power application for the time elapsed previously stored in the memory (not shown). When the switch 25 is pressed, the microcontroller 55 causes the microprocessor 551 to control the microwave-generating unit 50 (in particular, amplifier 52) so that the microwave-generating unit 50 emits microwaves from the electrode section 24.

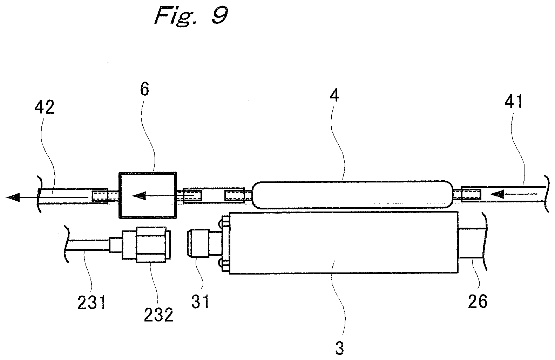

The present invention is not limited to the embodiments described above, but encompasses any and all embodiments within the intended scope of the present invention. For example, although the above embodiment discloses a structure in which cooling water is successively supplied to the cooling water bag 4, the present invention may also be structured such that cooling water is supplied only upon microwave irradiation. For example, as shown in Fig. 9, it is possible to provide a solenoid valve 6 between the cooling water bag 4 and the water discharge tube 42, and control the solenoid valve 6 by the microcontroller 55. In this structure, in response to the order to start microwave irradiation, the microcontroller 55 controls the start of the flow of cooling water by opening the solenoid valve 6. In response to the order to stop the microwave irradiation, the microcontroller 55 controls the stop of the flow of cooling water by closing the solenoid valve 6. This structure prevents unnecessary water flow that is not used for heat dissipation. The solenoid valve 6 may also be connected between the cooling water bag 4 and the water supply tube 41.

### Industrial Applicability

The present invention provides a mobile microwave surgical instrument that can be decreased in size, and thus can be easily carried.

### Reference Numerals

1 Microwave surgical instrument
2 Surgical instrument main body
24 Electrode section
3 Housing
4 Cooling water bag
5 Electronic circuit section
51 Microwave oscillator
52 Amplifier

## Claims

1. A microwave surgical instrument (1), comprising:
a grip (21) and
an electrode section (24) for emitting a microwave to a biological tissue so as to perform surgery on the biological tissue;
a microwave oscillator (51) for oscillating a microwave;
an amplifier (52), connected between the electrode section (24) and the microwave oscillator (51), for amplifying a microwave from the microwave oscillator (51) and transmitting the microwave to the electrode section (24);
a variable output matching circuit (53), connected between the amplifier (52) and the electrode section (24), for matching output impedance of the amplifier (52) and impedance of the biological tissue;
a detection circuit (54), connected between the variable output matching circuit (53) and the electrode section (24), for separately detecting a reflected power and an incident power between the amplifier (52) and the electrode section (24); and
controlling means (55) for controlling the variable output matching circuit (53) based on the values of the incident power and the reflected power detected by the detection circuit (54) such that the variable impedance of the biological tissue and the output impedance of the amplifier is matched, wherein
the grip (21) comprises, on its top end, an insertion unit (23) having the electrode section (24),
the electrode section (24) comprises a first electrode (241) and a second electrode (242) configured to come closer to each other to pinch the biological tissue, and wherein the microwave oscillator (51), the amplifier (52), the variable output matching circuit (53), the detection circuit (54) and the controlling means (55) are internally provided in the grip (21).

2. The microwave surgical instrument (1) according to claim 1, wherein the microwave surgical instrument (1) comprises a microwave-generating unit (50) containing the microwave oscillator (51) and the amplifier (52), the microwave-generating unit (50) being a semiconductor microwave-generating unit containing a semiconductor element for generating and amplifying a microwave.

3. The microwave surgical instrument (1) according to claim 1, further comprising:
a low-frequency constant current source (56) for supplying a low-frequency alternating current to the electrode section (24).

4. The microwave surgical instrument (1) according to claim 1, further comprising:
a housing (3) for storing an electronic circuit section (5) which comprises the microwave oscillator (51), the amplifier (52), the variable output matching circuit (53), the detection circuit (54) and the controlling means (55); and
a cooling water bag (4) into which cooling water is supplied, the cooling water bag (4) being provided near the housing (3) and configured to release heat from the housing (3) by the cooling water.

5. The microwave surgical instrument (1) according to claim 4, wherein the amount of cooling water supplied to the cooling water bag (4) is adjusted according to a timing of microwave irradiation.

6. The microwave surgical instrument (1) according to claim 4, wherein the cooling water is a physiological saline solution.

7. The microwave surgical instrument (1) according to claim 6, further comprising:
a water discharging path (42) for discharging the physiological saline solution from the cooling water bag (4) into the electrode section (24).

8. The microwave surgical instrument (1) according to claim 1, wherein the insertion unit (23) being detachable from the grip (21).

9. The microwave surgical instrument (1) according to claim 1, wherein the grip (21) has a portable size.

10. The microwave surgical instrument (1) according to claim 1, further comprising an inflexible coaxial cable for connecting the electrode section (24) and the amplifier (52).

11. The microwave surgical instrument (1) according to claim 1, wherein the variable output matching circuit (53) comprises:
an inductive element (531) having a first end connected to the amplifier (52) and a second end connected to the detection circuit (54); and
a first variable electrostatic capacitive element (532a) having a first end connected to the first end of the inductive element (531), and a second end that is grounded; and
a second variable electrostatic capacitive element (532b) having a first end connected to the second end of the inductive element (531), and a second end that is grounded,
and wherein the controlling means (55) is configured to adjust the electrostatic capacities of the first and second variable electrostatic capacitive elements (532a, 532b) according to the condition of the biological tissue that changes time-dependently during microwave irradiation.

## Patentansprüche

1. Chirurgisches Mikrowelleninstrument (1) mit:
einem Griff (21) und einem Elektrodenabschnitt (24) zum Emittieren einer Mikrowelle auf biologisches Gewebe, um eine Chirurgie bezüglich des biologischen Gewebes auszuführen;
einem Mikrowellenoszillator (51) zum Erzeugen einer oszillierenden Mikrowelle;
einem zwischen dem Elektrodenabschnitt (24) und dem Mikrowellenoszillator (51) geschalteten Verstärker (52) zum Verstärken einer vom Mikrowellenoszillator (51) zugeführten Mikrowelle und zum Übertragen der Mikrowelle zum Elektrodenabschnitt (24);
einer zwischen dem Verstärker (52) und dem Elektrodenabschnitt (24) geschalteten Ausgangsimpedanzanpassungsschaltung zum Anpassen der Ausgangsimpedanz des Verstärkers (52) an die Impedanz des biologischen Gewebes;
einer zwischen der Ausgangsimpedanzanpassungsschaltung (53) und dem Elektrodenabschnitt (24) geschalteten Erfassungsschaltung (54) zum separaten Erfassen einer reflektierten Leistung und einer eingestrahlten Leistung zwischen dem Verstärker (52) und dem Elektrodenabschnitt (24); und
einer Steuereinrichtung (55) zum Steuern der Ausgangsimpedanzanpassungsschaltung (53) basierend auf den Werten der eingestrahlten Leistung und der reflektierten Leistung, die durch die Erfassungsschaltung (54) erfasst werden, so dass die variable Impedanz des biologischen Gewebes und die Ausgangsimpedanz des Verstärkers aneinander angepasst werden, wobei
der Griff (21) an einem oberen Ende eine Einführeinheit (23) mit dem Elektrodenabschnitt (24) aufweist,
der Elektrodenabschnitt (24) eine erste Elektrode (241) und eine zweite Elektrode (242) aufweist, die dafür konfiguriert sind, eng aneinander angenähert zu werden, um das biologische Gewebe abzuklemmen, und
wobei der Mikrowellenoszillator (51), der Verstärker (52), die Ausgangsimpedanzanpassungsschaltung (53), die Erfassungsschaltung (54) und die Steuereinrichtung (55) im Inneren des Griffs (21) angeordnet sind.

2. Chirurgisches Mikrowelleninstrument (1) nach Anspruch 1, wobei das chirurgische Mikrowelleninstrument (1) eine Mikrowellenerzeugungseinheit (50) aufweist, die den Mikrowellenoszillator (51) und den Verstärker (52) aufweist, wobei die Mikrowellenerzeugungseinheit (50) eine Halbleiter-Mikrowellenerzeugungseinheit ist, die ein Halbleiterelement zum Erzeugen und Verstärken einer Mikrowelle aufweist.

3. Chirurgisches Mikrowelleninstrument (1) nach Anspruch 1, ferner mit:
einer niederfrequenten Konstantstromquelle (56) zum Zuführen eines niederfrequenten Wechselstroms zum Elektrodenabschnitt (24).

4. Chirurgisches Mikrowelleninstrument (1) nach Anspruch 1, ferner mit:
einem Gehäuse (3) zum Aufnehmen eines elektronischen Schaltungsabschnitts (5), der aufweist:
den Mikrowellenoszillator (51), den Verstärker (52), die Ausgangsimpedanzanpassungsschaltung (53), die Erfassungsschaltung (54) und die Steuereinrichtung (55); und
einen Kühlwasserbehälter (4), dem Kühlwasser zugeführt wird, wobei der Kühlwasserbehälter (4) in der Nähe des Gehäuses (3) angeordnet und dafür konfiguriert ist, durch das Kühlwasser Wärme vom Gehäuse (3) abzuleiten.

5. Chirurgisches Mikrowelleninstrument (1) nach Anspruch 4, wobei die dem Kühlwasserbehälter (4) zugeführte Kühlwassermenge gemäß einer Zeitsteuerung von Mikrowellenbestrahlung eingestellt wird.

6. Chirurgisches Mikrowelleninstrument (1) nach Anspruch 4, wobei das Kühlwasser eine physiologische Salzlösung ist.

7. Chirurgisches Mikrowelleninstrument (1) nach Anspruch 6, ferner mit:
einem Wasserableitpfad (42) zum Ableiten der physiologischen Salzlösung vom Kühlwasserbehälter (4) in den Elektrodenabschnitt (24).

8. Chirurgisches Mikrowelleninstrument (1) nach Anspruch 1, wobei die Einführeinheit (23) vom Griff (21) abnehmbar ist.

9. Chirurgisches Mikrowelleninstrument (1) nach Anspruch 1, wobei der Griff (21) eine tragbare Größe hat.

10. Chirurgisches Mikrowelleninstrument (1) nach Anspruch 1, ferner mit einem unflexiblen Koaxialkabel zum Verbinden des Elektrodenabschnitts (24) mit dem Verstärker (52).

11. Chirurgisches Mikrowelleninstrument (1) nach Anspruch 1, wobei die Ausgangsimpedanzanpassungsschaltung (53) aufweist:
ein induktives Element (531) mit einem mit dem Verstärker (52) verbundenen ersten Ende und einem mit der Erfassungsschaltung (54) verbundenen zweiten Ende; und
einem ersten variablen elektrostatischen kapazitiven Element (532a) mit einem mit dem ersten Ende des induktiven Elements (531) verbundenen ersten Ende und einem geerdeten zweiten Ende; und
einem zweiten variablen elektrostatischen kapazitiven Element (532b) mit einem mit dem zweiten Ende des induktiven Elements (531) verbundenen ersten Ende und einem geerdeten zweiten Ende,
und wobei die Steuereinrichtung (55) dafür konfiguriert ist, die elektrostatischen Kapazitäten des ersten und des zweiten variablen elektrostatischen kapazitiven Elements (532a, 532b) gemäß den Eigenschaften des

## Revendications

1. Instrument chirurgical à micro-ondes (1), comprenant :
une partie de préhension (21) et une section électrode (24) pour émettre une micro-onde vers un tissu biologique de manière à réaliser un acte chirurgical sur le tissu biologique ;
un oscillateur à micro-ondes (51) pour faire osciller une micro-onde ;
un amplificateur (52), relié entre la section électrode (24) et l'oscillateur à micro-ondes (51), pour amplifier une micro-onde provenant de l'oscillateur à micro-ondes (51) et transmettre la micro-onde à la section électrode (24) ;
un circuit de mise en correspondance de sortie variable (53), reliée entre l'amplificateur (52) et la section électrode (24), pour faire correspondre une impédance de sortie de l'amplificateur (52) et une impédance du tissu biologique ;
un circuit de détection (54), relié entre le circuit de mise en correspondance de sortie variable (53) et la section électrode (24), pour détecter séparément une puissance réfléchie et une puissance incidente entre l'amplificateur (52) et la section électrode (24); et
un moyen de commande (55) pour commander le circuit de mise en correspondance de sortie variable (53) sur la base des valeurs de la puissance incidente et de la puissance réfléchie détectées par le circuit de détection (54) de telle sorte que l'impédance variable du tissu biologique et l'impédance de sortie de l'amplificateur correspondent, dans lequel
la partie de préhension (21) comprend, sur son extrémité supérieure, une unité d'insertion (23) ayant la section électrode (24),
la section électrode (24) comprend une première électrode (241) et une seconde électrode (242) configurées pour se rapprocher l'une de l'autre afin de pincer le tissu biologique, et
dans lequel l'oscillateur à micro-ondes (51), l'amplificateur (52), le circuit de mise en correspondance de sortie variable (53), le circuit de détection (54) et le moyen de commande (55) sont disposés à l'intérieur de la partie de préhension (21).

2. Instrument chirurgical à micro-ondes (1) selon la revendication 1, dans lequel l'instrument chirurgical à micro-ondes (1) comprend une unité de génération de micro-ondes (50) contenant l'oscillateur à micro-ondes (51) et l'amplificateur (52), l'unité de génération de micro-ondes (50) étant une unité de génération de micro-ondes à semi-conducteurs contenant un élément semi-conducteur pour générer et amplifier une micro-onde.

3. Instrument chirurgical à micro-ondes (1) selon la revendication 1, comprenant en outre :
une source de courant constant basse-fréquence (56) pour apporter le courant alternatif basse-fréquence à la section électrode (24).

4. Instrument chirurgical à micro-ondes (1) selon la revendication 1, comprenant en outre :
un boîtier (3) pour stocker une section circuit électronique (5) qui comprend l'oscillateur à micro-ondes (51), l'amplificateur (52), le circuit de mise en correspondance de sortie variable (53), le circuit de détection (54) et le moyen de commande (55) ; et
un sac d'eau de refroidissement (4) dans lequel de l'eau de refroidissement est apportée, le sac d'eau de refroidissement (4) se situant près du boîtier (3) et étant configuré pour libérer la chaleur du boîtier (3) par l'eau de refroidissement.

5. Instrument chirurgical à micro-ondes (1) selon la revendication 4, dans lequel la quantité d'eau de refroidissement apportée au sac d'eau de refroidissement (4) est ajustée en fonction d'un moment d'irradiation aux micro-ondes.

6. Instrument chirurgical à micro-ondes (1) selon la revendication 4, dans lequel l'eau de refroidissement est une solution saline physiologique.

7. Instrument chirurgical à micro-ondes (1) selon la revendication 6, comprenant en outre :
un chemin de décharge d'eau (42) pour décharger la solution saline physiologique depuis le sac d'eau de refroidissement (4) dans la section électrode (24).

8. Instrument chirurgical à micro-ondes (1) selon la revendication 1, dans lequel l'unité d'insertion (23) peut être détachée de la partie de préhension (21).

9. Instrument chirurgical à micro-ondes (1) selon la revendication 1, dans lequel la partie de préhension (21) a une taille portative.

10. Instrument chirurgical à micro-ondes (1) selon la revendication 1, comprenant en outre un câble coaxial inflexible pour relier la section électrode (24) et l'amplificateur (52).

11. Instrument chirurgical à micro-ondes (1) selon la revendication 1, dans lequel le circuit de mise en correspondance de sortie variable (53) comprend :
un élément inductif (531) ayant une première extrémité reliée à l'amplificateur (52) et une seconde extrémité reliée au circuit de détection (54) ; et
un premier élément capacitif électrostatique variable (532a) ayant une première extrémité reliée à la première extrémité de l'élément inductif (531), et une seconde extrémité qui est reliée à la terre ; et
un second élément capacitif électrostatique variable (532b) ayant une première extrémité reliée à la seconde extrémité de l'élément inductif (531), et une seconde extrémité qui est reliée à la terre ;
et dans lequel le moyen de commande (55) est configuré pour ajuster les capacités électrostatiques des premier et second éléments capacitifs électrostatiques variables (532a, 532b) en fonction de l'état du tissu biologique qui change en fonction du temps durant l'irradiation aux micro-ondes
